Europäisches Patentamt

European Patent Office

Office européen des brevets

(19)

(11) **EP 0 726 241 B1**

(12) **EUROPÄISCHE PATENTSCHRIFT**

(45) Veröffentlichungstag und Bekanntmachung des Hinweises auf die Patenterteilung:
**17.09.1997 Patentblatt 1997/38**

(51) Int. Cl.$^6$: **C07C 11/09**, C07C 1/24, B01J 8/02

(21) Anmeldenummer: **95119273.1**

(22) Anmeldetag: **07.12.1995**

(54) **Verfahren zur Spaltung von Tertiärbutylalkohol in einer Reaktionsdestillationskolonne**

Process for cleavage of tertiary butyl alcohol in a reaction and distillation column

Procédé pour la scission de l'alcool tert.-butylique dans une colonne de distillation et de réaction

(84) Benannte Vertragsstaaten:
**AT BE DE ES FR GB IT NL**

(30) Priorität: **11.02.1995 DE 19504555**

(43) Veröffentlichungstag der Anmeldung:
**14.08.1996 Patentblatt 1996/33**

(73) Patentinhaber: **HÜLS AKTIENGESELLSCHAFT 45764 Marl (DE)**

(72) Erfinder:
- **Sakuth, Michael, Dr.**
  **D-45772 Marl (DE)**
- **Peters, Udo, Dr.**
  **D-45770 Marl (DE)**

(56) Entgegenhaltungen:
**US-A- 5 231 234**

EP 0 726 241 B1

**Beschreibung**

Die vorliegende Erfindung betrifft ein Verfahren zur Spaltung von Tertiärbutylalkohol (TBA) an sauren Ionenaustauscherharzen in einer Reaktionsdestillationskolonne. Das Verfahren dient der Gewinnung von Isobuten hoher Reinheit mit verbesserter Wirtschaftlichkeit.

Tertiärbutylalkohol (TBA) wird durch eine Reaktion von Isobuten mit Wasser gewonnen. Die sich an die TBA-synthese anschließende, endotherm verlaufende Spaltung des Alkohols dient der Gewinnung von Isobuten hoher Reinheit. Der Umweg über den Alkohol erfolgt wegen der sehr geringen Siedepunktsdifferenz bzw. wegen des sehr geringen Trennfaktors zwischen Isobuten und 1-Buten, wodurch eine direkte destillative Auftrennung isobutenhaltiger Raffinatströme unwirtschaftlich ist.

Die säurekatalysierte Spaltung (Dehydratisierung) von Tertiärbutylalkohol (TBA) zur Gewinnung von Isobuten hoher Reinheit ist ein bekanntes Verfahren. Bekannt sind hierbei zwei unterschiedliche Prozeßvarianten. Zum einen erfolgt die Spaltung in der Flüssigphase an sauren Ionenaustauscherharzen - beispielsweise in US 4 423 271 -. Zum anderen wird die Dehydratisierungsreaktion aber auch in der Gasphase an sauren Alumosilikatkatalysatoren - beispielsweise in JP 184961/86 - oder an sauren Aluminiumoxiden - beispielsweise in US 3 665 048 - durchgeführt. Die Abspaltung von Wasser aus Tertiärbutylalkohol an sauren Ionenaustauscherharzen in der Flüssigphase wird auch in einem Artikel von Health et al. ("Acid Resin Catalysis: The dehydration of t-butyl-Alcohol", AIChE J. 18 (2), 321 - 326, 1972) ausführlich beschrieben.

Bei der ersten Prozeßvariante, die in der Flüssigphase durchgeführt wird, ist zu beachten, daß prinzipiell nur kleine TBA-Umsätze per pass gefahren werden können. Dies begründet sich im flüssig-flüssig-Gleichgewicht des Ternärsystems TBA/Wasser/Isobuten, das bereits bei kleinen Isobutenkonzentrationen zur Zweiphasigkeit neigt. Bei hohen TBA-Umsätzen würde sich eine TBA-reiche, organische Phase sowie eine TBA-ärmere, wäßrige Phase ausbilden. Aufgrund der üblichen, hydrophilen Eigenschaften der verwendeten Katalysatorsysteme würde der Katalysator bevorzugt die wäßrige Phase aufnehmen, und die Spaltungsreaktion würde zum Erliegen kommen. Höhere Umsätze lassen sich nur durch die Anwendung hoher Kreislaufmengen mit zwischengeschalteter, destillativer Aufarbeitung zur Abtrennung des Isobutens erzielen. Weiterer Problempunkt bei dieser Prozeßvariante ist die in der homogenen Flüssigphase gelöste Menge an Isobuten, die wiederum nicht so gering ist, daß Folgereaktionen dieser Molekülspezies in der Reaktionszone unterbleiben. Wichtigste Reaktionen dieser Art sind die säurekatalysierte Dimerisierung und Oligomerisierung. Aus diesem Grunde findet man neben dem gewünschten Zielprodukt Isobuten auch $C_8$- sowie $C_{12}$-Komponenten. Bei den unerwünschten $C_8$-Molekülen handelt es sich um 2,4,4-Trimethyl-1-penten sowie 2,4,4-Trimethyl-2-penten.

Bei der zweiten Prozeßvariante, bei der man die Spaltungsreaktion in der Gasphase durchführt, treten wegen der hier verfahrensbedingt hohen Temperaturen und hohen Isobutenkonzentrationen ebenfalls die Probleme der Dimerisierung bzw. Oligomerisierung des gebildeten Isobutens zu unerwünschten Folgeprodukten auf. Indem man den dampfförmigen Eduktstrom mit Inertgas verdünnt, versucht man üblicherweise, diese Reaktionen zu verhindern. Hierdurch wird ein zusätzlicher Aufarbeitungsaufwand verursacht.

Beide Prozeßvarianten werden in herkömmlichen (Festbett-)Reaktoren durchgeführt.

In Reaktionsdestillationskolonnen ist die Anordnung des Katalysators sowohl im Sumpf der Kolonne als auch oberhalb des Sumpfes im gasförmigflüssigen Bereich bekannt.

US-5 231 234 beschreibt ein zweistufiges Verfahren zur Herstellung von Ethern aus tertiären Alkoholen. Bei der Herstellung von Methyl-tertiärbutylether besteht der erste Verfahrensschritt in einer Spaltung von reinem TBA in Isobuten und Wasser in einer Reaktionsdestillationskolonne an einem sauren Ionenaustauscherharz als Katalysator. Die Spaltung erfolgt bei nur 74 °C bis 93 °C, obwohl höhere Temperaturen die Spaltung begünstigen würden, um einer Oligomerisierung des entstehenden Isobutens entgegenzuwirken. Zu diesem Zweck wird auch die Reaktionszone stets durch entstehendes Reaktionswasser ausreichend naß gehalten. Das nach fraktionierter Destillation zur Abtrennung des Wasser über Kopf abgezogene Isobuten wird im zweiten Verfahrensschritt in einer weiteren Reaktionsdestillationskolonne mit Methanol umgesetzt.

In EP 0 302 336 B1 wird die Spaltung von Alkyltertiäralkylether in einer Reaktionsdestillationskolonne dargestellt, wobei als Katalysator ein saurer Kationenaustauscher im Sumpf der Kolonne, also in der Flüssigphase, eingesetzt wird. Diese Reaktion dient im Falle der Spaltung von Methyl-tertiär-butylether (MTBE) oder Ethyl-tertiär-butyl-ether (ETBE) ebenfalls der Gewinnung von Isobuten hoher Reinheit. Aufgrund der thermodynamischen Reaktionsgleichgewichtslage ist aber eine Spaltung von Tertiärbutylalkohol für die Isobutengewinnung generell wirtschaftlicher.

Der vorliegenden Erfindung liegt daher die Aufgabe zugrunde, ein Verfahren zur Durchführung der Spaltung von Tertiärbutylalkohol in einer Reaktionsdestillationskolonne zur Gewinnung von Isobuten hoher Reinheit zu entwickeln, das höhere TBA-Umsätze per pass als die herkömmlichen Verfahren ermöglicht und gleichzeitig eine geringe Folgeproduktbildung aus Isobuten aufweist.

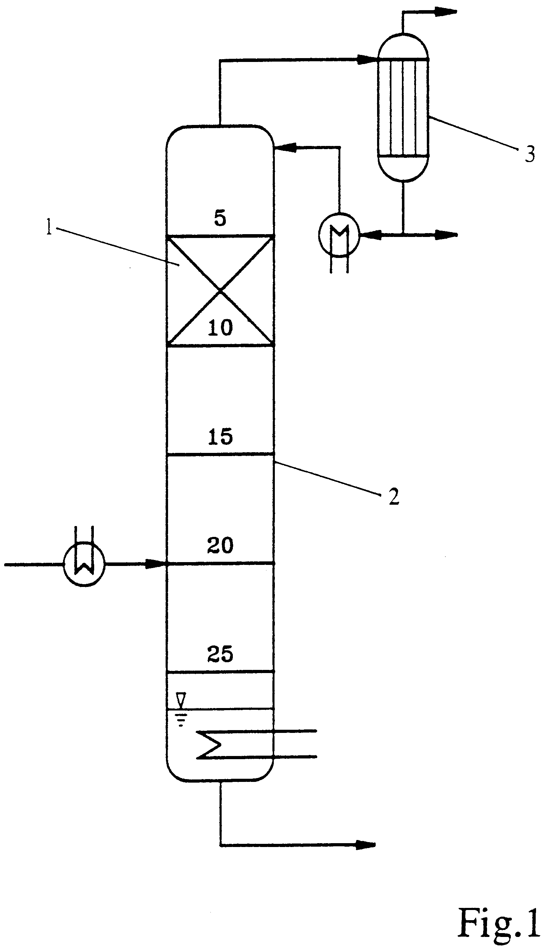

Die Aufgabe wird erfindungsgemäß gelöst durch das im Patentanspruch 1 gekennzeichnete Verfahren, das in einer bevorzugten Ausführung schematisch in Figur 1 dargestellt ist. Als Katalysator 1 für die Spaltung von TBA in Isobuten und Wasser dient ein saures Ionenaustauscherharz, das - bevorzugt innerhalb einer Destillationspackung - oberhalb des Sumpfes im gasförmig-flüssigen Bereich angeordnet ist. Das zumindest TBA, Isobuten und Wasser enthaltende

2

Reaktionsprodukt wird am Kopf der Reaktionsdestillationskolonne 2 abgezogen und dem mit dem Kopf der Kolonne gekoppelten Dephlegmator 3 zugeführt. Durch teilweise Kondensation wird das Reaktionsprodukt im Dephlegmator in einen gasförmigen, Isobuten-reichen Teil, der das Verfahrenswertprodukt darstellt, und einen flüssigen, Edukt-reichen Teil getrennt, der zumindest zum Teil als Rücklauf auf den Kolonnenkopf aufgegeben wird. Der Einsatz des Dephlegmators dient an dieser Stelle der Überwindung der Zweiphasigkeit im Flüssig-Flüssig-Gleichgewicht des Ternärsystems TBA/Wasser/Isobuten, die bei einer vollständigen Kondensation des Reaktionsproduktes (also auch des Isobutens) auftreten würde. Im Sumpf der Kolonne wird ein Wasser-reiches Sumpfprodukt abgezogen. Wie in Figur 1 bereits dargestellt, erfolgt der Zulauf des zumindest TBA-reichen Edukts bevorzugt unterhalb der Katalysatorpackung. Üblicherweise dienen TBA/Wasser-Gemische als Edukt.

Als Katalysator kommen stark saure Kationenaustauscherharze, beispielsweise solche auf Basis von Styrol-Divinylbenzol-Harzen, Phenol-Formaldehyd-Harzen oder Cumaron-Inden-Harzen, in Frage, wobei die aromatischen Kerne Sulfonsäuregruppen tragen. Die Kationenaustauscherharze werden in ihrer protonenhaltigen Form eingesetzt.

Ein grundlegender Vorteil des erfindungsgemäßen Verfahrens besteht darin, daß bei einer Realisierung der Tertiärbutylalkoholspaltung in einer Reaktionsdestillationskolonne eine günstige Beeinflussung der endotherm verlaufenden Gleichgewichtsreaktion:

$$TBA \leftrightarrow Wasser + Isobuten$$

durch destillatives Entfernen des Reaktionspartners Isobuten resultiert, wodurch höhere TBA-Umsätze begünstigt werden.

In überraschend vorteilhafter Weise ermöglicht die erfindungsgemäße Anordnung der Kationenaustauscherpackung oberhalb des Sumpfes sowie die Kopplung der Reaktionsdestillationskolonne mit einem Dephlegmator eine Überwindung der Zweiphasigkeit im Flüssig-Flüssig-Gleichgewicht des Ternärsystems TBA/Wasser/Isobuten. Durch den Einsatz eines kaum isobutenhaltigen Rücklaufs wird die sonst üblicherweise eintretende Abnahme des Bodenwirkungsgrades bei Zweiphasigkeit überwunden. Im allgemeinen beträgt der Isobutengehalt im Rücklauf weniger als 5 Gew.-% und der TBA-Gehalt mehr als 80 Gew.-%.

Dadurch, daß das am Dephlegmator flüssig anfallende, TBA-reiche Destillat bevorzugt möglichst vollständig zurückgeführt wird, erzielt man außerdem eine vergleichsweise hohe TBA-Konzentration im Reaktionsteil der Kolonne, zumal wenn dieser - wie bevorzugt - in Kolonnenkopfnähe angeordnet ist. Weiterhin wird hierdurch auch die mittlere Verweilzeit des Alkohols in der Reaktionszone erhöht. Ordnet man zusätzlich, wie es die bevorzugte Ausführung in Figur 1 zeigt, die Destillationsreaktionspackung oberhalb des Zulaufes im oberen Teil der Reaktionsdestillationskolonne an und fährt man ein TBA/Wasser-Gemisch beliebiger Zusammensetzung in die Kolonne, so erlaubt der Verstärkerteil bis zum Beginn der Reaktionszone bereits eine Auftrennung des TBA/Wasser-Gemisches bis zum azeotropen Punkt und damit die maximal mögliche TBA-Anreicherung. Damit wird gewährleistet, daß immer ein möglichst TBA-reiches Gemisch den Reaktionsteil der Reaktionsdestillationskolonne erreicht. Durch dieses Vorgehen läßt sich insgesamt der TBA-Umsatz so deutlich erhöhen, daß - im Grenzfall der vollständigen Rückführung des flüssig anfallenden Destillats - am Dephlegmator fast reines Isobuten abgezogen werden kann, während im Sumpf fast reines Wasser die Kolonne verläßt. Üblicherweise lassen sich TBA-Umsätze von über 70 % bezogen auf den TBA-Gehalt im Eduktstrom erzielen. Der Gehalt an Isobuten im Wertprodukt, das den Dephlegmator gasförmig verläßt, beträgt im allgemeinen über 97 Gew.-%, wobei die restlichen Bestandteile (in der Hauptsache) TBA und Wasser sind. Eine bevorzugt destillative Aufarbeitung des Produkts zur Abtrennung des Isobutens ist bei Bedarf einfach bzw. mittels weniger Trennstufen möglich.

Weiterhin wird durch die beim erfindungsgemäßen Verfahren erzielte niedrige Konzentration von Isobuten in der Flüssigphase die Bildung von unerwünschten Folgeprodukten durch Dimerisierung bzw. Oligomerisierung von Isobuten in der Reaktionszone weitgehend verhindert. Hierzu trägt auch die bevorzugte Anordnung der Katalysatorpackung oberhalb des Zulaufs in Kolonnenkopfnähe bei.

Die Zweiphasigkeit im Flüssig-Flüssig-System TBA/Wasser/Isobuten bei höheren Isobutenkonzentrationen wird zudem durch die erfindungsgemäß bevorzugte Anordnung der Katalysatorpackung im oberen Kolonnenteil bzw. oberhalb des Zulaufes und durch die Anwendung eines Dephlegmators am Kolonnenkopf überwunden. Insbesondere läßt sich dadurch - ohne Anwendung äußerer Kreislaufströme - eine höhere Raum-Zeit-Ausbeute wie auch ein hoher Umsatzgrad erzielen. Das erfindungsgemäße Verfahren erlaubt damit eine wirtschaftlichere Gewinnung von Isobuten hoher Reinheit.

Die Reaktionsdestillationskolonne wird bevorzugt im Druckbereich von 0,1 bis 6,0 bar$_{abs}$ bei Temperaturen bis maximal 150 °C, besonders bevorzugt bei einem Druck zwischen 2,0 und 4,0 bar$_{abs}$ und einer Temperatur bis 130 °C, betrieben.

Der besonders bevorzugte Arbeitsbereich der Reaktionsdestillationskolonne bezüglich des Druckes liegt bei etwa 3,0 bar$_{abs}$. Damit kann gewährleistet werden, daß die maximale Temperatur, mit der der Katalysator, das Kationenaustauscherharz, belastet wird, bei etwa 120 °C liegt. Bei dieser Temperatur ist noch nicht mit einer deutlichen Abspaltung sulfonsaurer Gruppen von der Harzoberfläche zu rechnen. Die Katalysatoraktivität bleibt somit über einen längeren Zeitraum erhalten.

Das folgende Ausführungsbeispiel soll die vorliegende Erfindung verdeutlichen.

Beispiel:

Die TBA-Spaltung wird in einer Druckkolonne mit Glockenböden durchgeführt. Die Kolonne, die adiabat betrieben wird, besitzt folgende Abmessungen und Anordnungen:

- Innendurchmesser: 80 mm
- Anordnung:

    - insgesamt 5 Schüsse mit jeweils 5 Glockenböden
    - 2. oberer Schuß = Reaktionsschuß, ausgestattet mit einer Metallgewebepackung, die mit dem Katalysator (saurer Kationenaustauscher) gefüllt ist. Es wurden experimentelle Destillationsreaktionspackungen verwendet, wie sie beispielsweise im Patent US 5 073 236 beschrieben werden.

Das Edukt, bestehend aus 87 Gew.-% TBA und 13 Gew.-% Wasser, wird auf dem Boden 20 der Kolonne mit einer Temperatur von 90 °C unterkühlt hineingeführt.

Das Kopfprodukt wird abgezogen und im angeschlossenen Dephlegmator auf ca. 35 °C abgekühlt. Bei dieser Temperatur und einem Kolonnendruck von 2,8 bar$_{abs.}$ ist gewährleistet, daß sich das Isobuten bevorzugt in der Dampfphase befindet, während der Gehalt an TBA und Wasser in dieser Phase gering ist. Eine deutlich niedrigere Temperatur würde hier eine merkliche Isobutenkondensation hervorrufen, die zur Zweiphasigkeit im Flüssig-Flüssig-System und damit im Rücklauf führt. Es wird somit eine sehr isobutenreiche Dampfphase (97 Gew.-% Isobuten) aus dem Dephlegmator abgezogen, die das Wertprodukt darstellt. Ferner wird ein Teil des Destillats aus dem Prozeß ausgeschleust. Der Rest wird auf ca. 70 °C erwärmt und auf den Kolonnenkopf aufgegeben.

Tabelle 1 gibt eine Übersicht über die Massenströme und ihre Zusammensetzung. Das Rücklaufverhältnis beträgt 1,7.

Tabelle 1

| Massenströme und ihre Hauptbestandteile (gerundet) | | | | |
|---|---|---|---|---|
| | kg/h | Gew.-% (mol-%) TBA | Gew.-% (mol-%) Wasser | Gew.-% (mol-%) Isobuten |
| Edukt | 2,31 | 87 (62) | 13 (38) | 0 ( 0) |
| Wertprodukt | 0,72 | 2 ( 1) | 1 ( 3) | 97 (96) |
| abgezogenes Destillat | 0,88 | 82 (57) | 13 (38) | 5 ( 5) |
| Sumpfprodukt | 0,71 | 40 (14) | 60 (86) | 0 ( 0) |

Durch Variation des Rücklaufverhältnisses läßt sich der TBA-Umsatz deutlich bis auf weit über 90 %, bezogen auf den TBA-Gehalt im Eduktstrom, steigern.

**Patentansprüche**

1. Verfahren zur Spaltung von Tertiärbutylalkohol in einer Reaktionsdestillationskolonne, gekennzeichnet durch

    - das Durchführen der Reaktion an einem sauren Ionenaustauscherharz als Katalysator, das oberhalb des Sumpfes angeordnet ist,
    - das teilweise Kondensieren des Kopfproduktes der Reaktionsdestillationskolonne in einem Dephlegmator, der mit dem Kopf der Reaktionsdestillationskolonne verbunden ist, und
    - das Aufgeben von zumindest einem Teil des flüssig anfallenden, eduktreichen Destillats aus dem Dephlegmator auf den Kolonnenkopf als Rücklauf.

2. Verfahren nach Anspruch 1,
   gekennzeichnet durch

- das Zuführen des Edukts unterhalb der Katalysatorpackung.

3. Verfahren nach Anspruch 1 oder 2,
   dadurch gekennzeichnet,
   daß das flüssig anfallende, eduktreiche Destillat möglichst vollständig auf den Kopf der Destillationskolonne zurückgeführt wird.

4. Verfahren nach einem der Ansprüche 1 bis 3,
   gekennzeichnet durch

   - einen Druck in der Reaktionsdestillationskolonne zwischen 0,1 und 6,0 bar$_{abs}$ bei einer Temperatur bis 150 °C.

5. Verfahren nach Anspruch 4,
   gekennzeichnet durch

   - einen Druck in der Reaktionsdestillationskolonne zwischen 2,0 und 4,0 bar$_{abs}$ bei einer Temperatur bis 130 °C.

6. Vorrichtung zur Spaltung von Tertiärbutylalkohol gemäß Anspruch 1,
   gekennzeichnet durch

   - die Kopplung des Kopfs der Reaktionsdestillationskolonne mit einem Dephlegmator.

7. Verwendung des Verfahrens gemäß Anspruch 1 zur Spaltung von Tertiärbutylalkohol und zur Gewinnung von Isobuten hoher Reinheit.

## Claims

1. A process for cracking tertiary butyl alcohol in a reaction distillation column, characterized in that

   - the reaction is carried out over an acid ion exchange resin as catalyst, which is arranged above the liquid phase zone,
   - the top product of the reaction distillation column is partially condensed in a dephlegmator which is connected to the top of the reaction distillation column, and
   - at least a part of the liquid, starting material-rich distillate from the dephlegmator is returned to the top of the column as runback.

2. A process according to claim 1, characterized in that

   - the starting material is fed in below the catalyst pack.

3. A process according to claim 1 or 2, characterized in that the liquid, starting material-rich distillate is recirculated as completely as possible to the top of the distillation column.

4. A process according to any one of claims 1 to 3, characterized by

   - a pressure in the reaction distillation column of from 0.1 to 6.0 bar$_{abs}$ at a temperature of up to 150 °C.

5. A process according to claim 4, characterized by

   - a pressure in the reaction distillation column of from 2.0 to 4.0 bar$_{abs}$ at a temperature of up to 130 °C.

6. An apparatus for cracking tertiary butyl alcohol according to claim 1, characterized in that

   - the top of the reaction distillation column is coupled with a dephlegmator.

7. Use of the process according to claim 1 for cracking tertiary butyl alcohol and for isolating isobutene of high purity.

**Revendications**

1. Procédé de décomposition d'alcool tert butylique dans une colonne de distillation à réaction, caractérisé par

   • la réalisation de la réaction auprès d'une résine échangeuse d'ions acide comme catalyseur, disposée au-dessus du pied de colonne,
   • la condensation partielle du produit de tête de la colonne de distillation à réaction dans un déflegmateur connexe à la tête de la colonne de distillation à réaction, et
   • l'introduction d'au moins une partie du distillat riche en réactifs obtenu sous forme liquide depuis le déflegmateur vers la tête de colonne comme reflux.

2. Procédé selon la revendication 1 caractérisé par l'alimentation en réactif en-dessous du garnissage du catalyseur.

3. Procédé selon la revendication 1 ou 2, caractérisé en ce que le distillat riche en réactif obtenu sous forme liquide est reconduit le plus complètement possible vers la tête de la colonne de distillation.

4. Procédé selon une des revendications 1 à 3, caractérisé par une pression dans la colonne de distillation à réaction comprise entre 0,1 et 6,0 bars$_{abs}$ pour une température jusqu'à 150°C.

5. Procédé selon la revendication 4, caractérisé par une pression dans la colonne de distillation à réaction comprise entre 2,0 et 4,0 bars$_{abs}$ pour une température jusqu'à 130°C.

6. Dispositif pour la décomposition d'alcool tert butylique selon la revendication 1, caractérisé par le couplage de la tête de la colonne de distillation à réaction avec un déflegmateur.

7. Utilisation du procédé selon la revendication 1 pour la décomposition d'alcool tert butylique et pour l'obtention d'isobutène de grande pureté.

Fig.1